# EUROPEAN PATENT SPECIFICATION

(11) **EP 3 565 570 B1**
(45) Date of publication and mention of the grant of the patent: **03.07.2024**
(21) Application number: 18701798.3
(22) Date of filing: 04.01.2018
(51) Int. Cl.: A61K 38/00, A61K 38/03, C07K 14/47

(54) **COMPOSITION**
ZUSAMMENSETZUNG
COMPOSITION

(30) Priority: 04.01.2017 GB 201700095
(43) Date of publication of application: 13.11.2019
(73) Proprietor: Worg Pharmaceuticals (Zhejiang) Co., Ltd., Huzhou, Zhejiang (CN)
(72) Inventor: MARTIN, Keith, Chepstow Gwent NP16 5UH (GB); JANSSON, Liselotte, Chepstow Gwent NP16 5UH (GB)
(74) Representative: D Young & Co LLP
(86) International application number: PCT/IB2018/050062
(87) International publication number: WO 2018/127829

(56) References cited:
- WO-A1-02/077025
- WO-A2-02/16410
- WO-A2-2009/056833
- WO-A2-2014/160465
- GRAZYNA ADAMUS ET AL: "Treatment of Autoimmune Anterior Uveitis with Recombinant TCR Ligands", INVESTIGATIVE OPTHALMOLOGY & VISUAL SCIENCE, vol. 47, no. 6, 1 June 2006 (2006-06-01), page 2555, XP055454346, US ISSN: 1552-5783, DOI: 10.1167/iovs.05-1242
- GRAZYNA ADAMUS ET AL: "Importance of cryptic myelin basic protein epitopes in the pathogenicity of acute and recurrent anterior uveitis associated with EAE", JOURNAL OF NEUROIMMUNOLOGY, vol. 113, no. 2, 1 February 2001 (2001-02-01), pages 212-219, XP055046367, ISSN: 0165-5728, DOI: 10.1016/S0165-5728(00)00439-2

## Description

### FIELD OF THE INVENTION

The present invention relates to a composition which comprises a peptide or several peptides derived from Myelin Basic Protein (MBP). The composition or peptide may be useful in the prevention and/or suppression of autoimmunity, for example the prevention and/or suppression of effector T helper cells, which is useful in the treatment and/or prevention of uveitis. The invention also relates to corresponding methods of treatment and uses of the peptide in the manufacture of a medicament for the treatment and/or prevention of uveitis.

### BACKGROUND TO THE INVENTION

Uveitis describes a group of diseases associated with inflammation of the uvea. The uvea is a region of the eye located between the sclera and the retina, and includes the iris, ciliary body and choroid. The uvea provides most of the blood supply to the retina. The associated diseases are not restricted to those affecting the uvea directly, and adjacent structures such as the retina, optic nerve, lens, vitreous and sclera can be affected in manifestations of uveitis.

All forms of uveitis are characterised by an inflammatory cellular infiltrate, commonly visualised using a biomicroscope. In 2010, it was estimated that 285 million people were visually impaired; of these, 39 million were blind and it was approximated that 10% of the cases were due to uveitis. (Global data on visual impairments, The World Health Report, WHO (2010) http://www.who.int/blindness/GLOBALDATAFINALforweb.pdf)

Present treatments for uveitis include the use of glucocorticoid steroids and other broad immunosuppressive agents such as methotrexate. There is, however, a need in the art for alternative treatments for uveitis. The present invention addresses this need.

Adamus et al. Invest Opthalmol Vis Sci. 2006, 47, 2555-2561 disclosed treatment of autoimmune anterior uveitis with recombinant TCR ligands.

WO 2009/056833 discloses the use of the MBP 30-44, 83-99, 131-145, and 140-154 peptides to treat patients with secondary multiple sclerosis while monitoring the effects on associated optic neuritis.

### SUMMARY OF THE INVENTION

The present inventors have identified a number of peptides derived from Myelin Basic Protein (MBP) which may be useful in the prevention and/or treatment of uveitis.

The present Examples demonstrate that in an *in vivo* model of uveitis the MBP peptides according to the invention can significantly reduce the clinical signs of uveitis.

Thus, the present invention provides a composition which comprises a myelin basic protein peptide selected from the following:
MBP 30-44 (SEQ ID NO: 1);
MBP 83-99 (SEQ ID NO:2);
MBP 131-145 (SEQ ID NO:3); and
MBP 140-154 (SEQ ID NO:4)
for use in treating or preventing uveitis in a subject.

In one aspect the composition for use in treating or preventing uveitis in a subject may comprise MBP 30-44 (SEQ ID NO:1), 83-99 (SEQ ID NO:2), 131-145 (SEQ ID NO:3) and 140-154 (SEQ ID NO:4), i.e. the composition may comprise a "cocktail" of the four peptides.

The composition for use in treating or preventing uveitis in a subject may consist essentially of MBP 30-44 (SEQ ID NO:1), 83-99 (SEQ ID NO:2), 131-145 (SEQ ID NO:3) and 140-154 (SEQ ID NO:4).

Described herein is a method for treating or preventing uveitis in a subject, wherein said method comprises the step of administration of a composition as described herein to the subject.

It has been found that the peptides binds several MHC class II molecules including HLA-DR and -DQ molecules and the combined use of these Apitopes provides more widespread cover of the different Major Histocompatibility Complex (MHC) haplotypes seen in uveitis patients than therapy with a single peptide.

The method of treatment or prevention may involve administration of the composition to, for example, a HLA-DQ6 or HLA-DR2 positive subject or subject expressing any other HLA type, for example DRB1, DRB3, DRB5, DQA1 or DQB1.

Described herein is use of the composition as described herein in the manufacture of a medicament for treating or preventing uveitis in a subject.

The invention also encompasses use of a composition as described herein in treating or preventing uveitis in a subject.

The present invention also provides a kit which comprises a myelin basic protein peptide selected from the following:
MBP 30-44 (SEQ ID NO: 1);
MBP 83-99 (SEQ ID NO:2);
MBP 131-145 (SEQ ID NO:3); and
MBP 140-154 (SEQ ID NO:4)
for use in the treatment of prevention of uveitis in a subject. In one aspect the composition comprises:
MBP 30-44 (SEQ ID NO:1);
MBP 83-99 (SEQ ID NO:2);
MBP 131-145 (SEQ ID NO:3); and
MBP 140-154 (SEQ ID NO:4) wherein said MBP peptides may be for simultaneous, separate or sequential administration.

### LIST OF FIGURES

**Figure 1****:** Clinical Scores of EAU (posterior uveitis). Data are presented as Mean ± SEM.
**Figure 2****:** Representative TEFI Images. (A) Score 0. (B): Score 1. (C) Score 2, engorged vessels. (D) Score 3. (E) Score 4. (F) Score 5. (G) Score 6.
**Figure 3****:** Clinical Scores of EAU (anterior uveitis). Data are presented as Mean ± SEM. Statistical significances: * p < 0.05, ** p < 0.01, *** p < 0.001, **** p < 0.0001.
**Figure 4****: Representative TEFI Images.** Cumulative scores of 1 - 10 due to enlarged blood vessels, increased vascularisation and infiltration.
**Figure 5****: Clinical Scores of EAU (posterior uveitis), huMBP and IRBP-immunised groups.** Data are presented as Mean ± SEM.
**Figure 6****: Clinical Scores of EAU (posterior uveitis), Area Under the Curve (AUC).** Data are presented as Mean ± SEM. Statistical significance: ***p < 0.001.
**Figure 7****: Anterior uveitis clinical scores (A.) and area under curve analysis (AUC) of anterior uveitis clinical scores (B.) in the huMBP** + **IRBP model.** Data are presented as mean ± SEM. Statistical significance: *, p < 0.05 and ****, p < 0.0001 for Cyclosporin A vs. vehicle; ^{#}, p < 0.05 and ^{####}, p < 0.0001 for ATX peptides vs. vehicle. Bold ticks on the x axis represent ATX peptides treatments after Day 0. Grey shaded area represents time course of Cyclosporin A treatment.

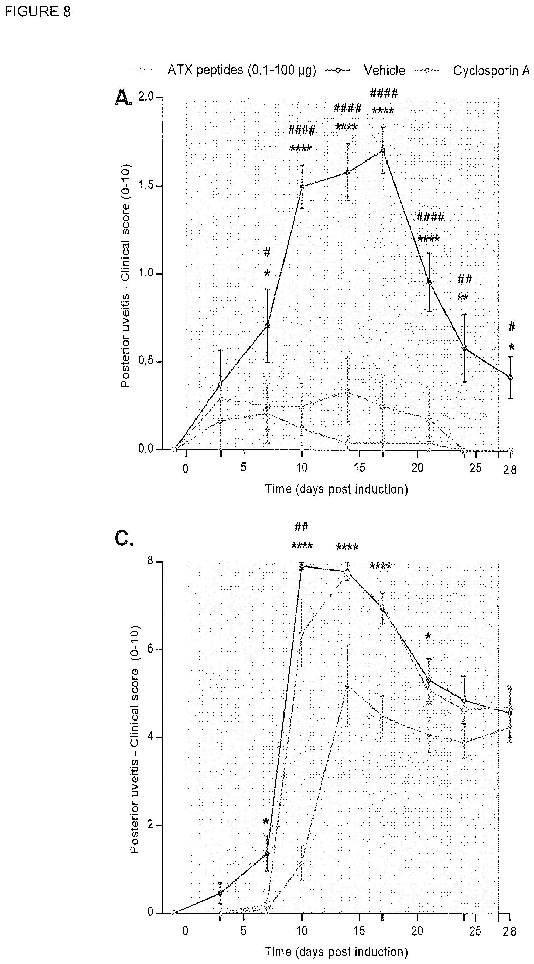

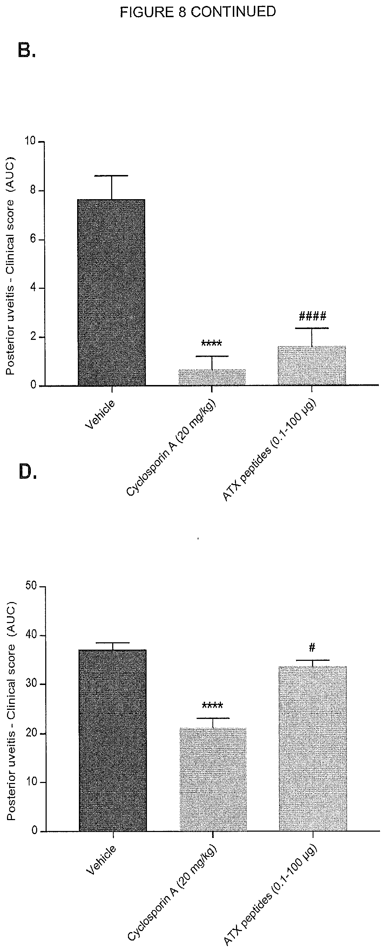

**Figure 8****: Posterior uveitis clinical scores (A.) and area under the curve analysis (AUC) of posterior uveitis clinical scores (B.) in the huMBP model. Posterior uveitis clinical scores (C.) and AUC analysis of posterior uveitis clinical scores (D.) in the huMBP** + **IRBP model.** Data are presented as mean ± SEM. Statistical significance: *, p < 0.05, **, p < 0.01 and ****, p < 0.0001 for Cyclosporin A vs. vehicle; ^{#}, p < 0.05, ^{##}, p < 0.01 and ^{####}, p < 0.0001 for ATX peptides vs. vehicle. Bold ticks on the x axis represent ATX peptides treatments after Day 0. Grey shaded area represents time course of Cyclosporin A treatment.
**Figure 9****: Posterior uveitis onset (%) in the huMBP (A.) and huMBP + IRBP (B.) models.** Statistical significance: ****, p < 0.0001 for Cyclosporin A *vs*. vehicle; ^{#}, p < 0.05 for ATX peptides vs. vehicle. Bold ticks on the x axis represent ATX peptides treatments after Day 0. Grey shaded area represents time course of Cyclosporin A treatment.

### DETAILED DESCRIPTION

### MYELIN BASIC PROTEIN

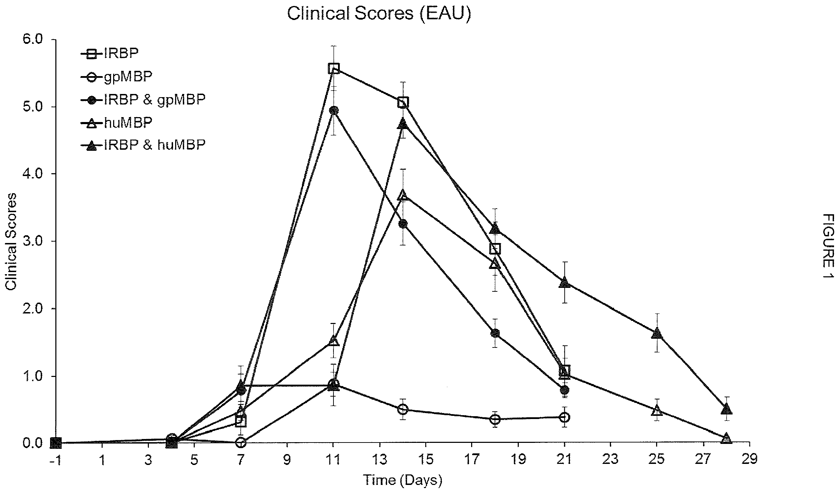

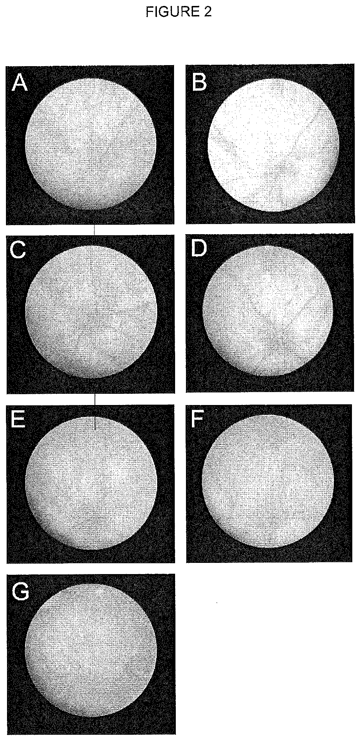

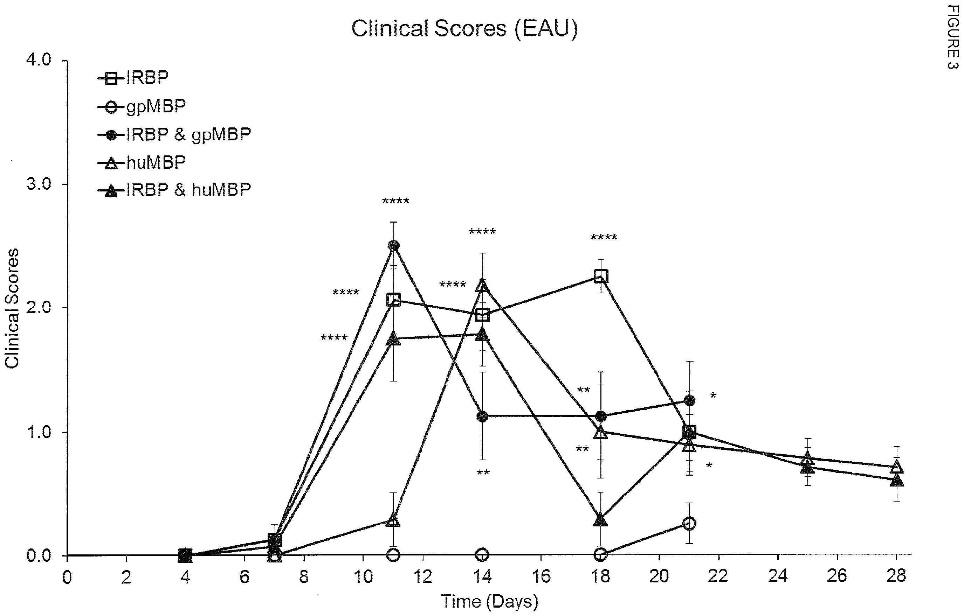

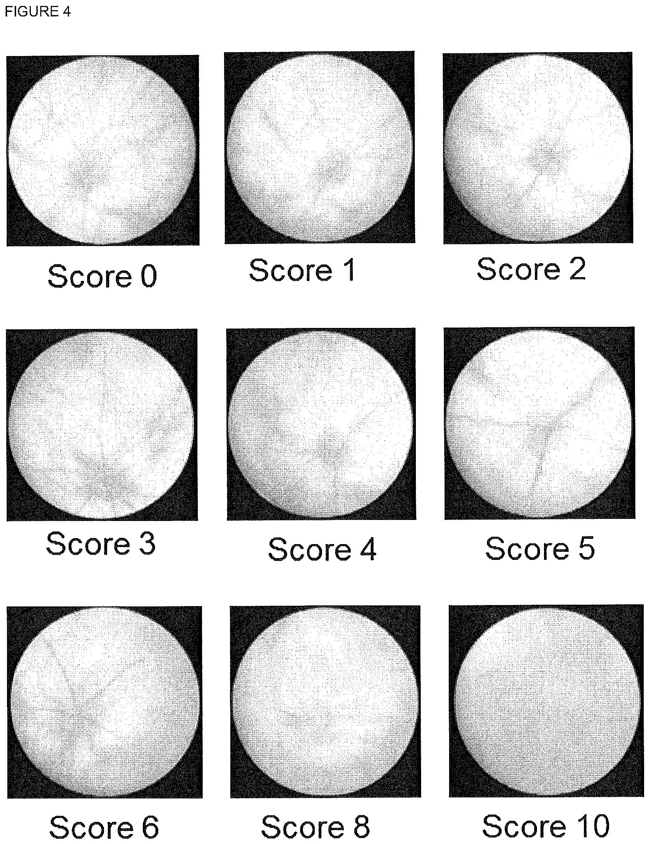

Myelin basic protein (MBP) is a protein isolatable from human brain white matter. The mature protein has 170 amino acids and the sequence is widely available in the literature (see for example: Chou et al (1986) J. Neurochem. 46:47-53, Figure 1; Kamholz et al (1986), PNAS 83:4962-4966, Figure 2; US Patent No. 5,817,629, SEQ ID NO: 1; Roth et al (1987), J. Neurosci. Res. 17:321-328, Figure 4; Medeveczky et al (2006), FEBS Letters 580:545-552, Figure 3B). Alternative splice variants/isoforms of MBP may be produced due to alternative splicing of MBP (see e.g. Kamholz *supra*)*.*

The composition for use according to the present invention comprises an MBP peptide selected from:
MBP 30-44 (SEQ ID NO: 1);
MBP 83-99 (SEQ ID NO:2);
MBP 131-145 (SEQ ID NO:3); and
MBP 140-154 (SEQ ID NO:4).

The composition may comprise MBP 30-44 (SEQ ID NO:1), 83-99 (SEQ ID NO:2), 131-145 (SEQ ID NO:3) and 140-154 (SEQ ID NO:4).

The composition may consist essentially of MBP 30-44 (SEQ ID NO:1), 83-99 (SEQ ID NO:2), 131-145 (SEQ ID NO:3) and 140-154 (SEQ ID NO:4).

The peptides present in the composition may consist of MBP 30-44 (SEQ ID NO:1), 83-99 (SEQ ID NO:2), 131-145 (SEQ ID NO:3) and 140-154 (SEQ ID NO:4). That is, no other MBP peptides are present. The MBP peptides may therefore consist of SEQ ID Nos:1 to 4.

By "consisting of" is meant including, and limited to, whatever follows the phrase "consisting of". Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they affect the activity or action of the listed elements.

### PEPTIDES

The peptides that may be used in the compositions and kits for use according to the present invention are as follows:
MBP 30-44 (SEQ ID NO: 1):
   H-Pro-Arg-His-Arg-Asp-Thr-Gly-Ile-Leu-Asp-Ser-Ile-Gly-Arg-Phe-NH2
MBP 83-99 (SEQ ID NO:2):
   H-Glu-Asn-Pro-Val-Val-His-Phe-Phe-Lys-Asn-Ile-Val-Thr-Pro-Arg-Thr-Pro-NH2
MBP 131-145 (SEQ ID NO:3):
   H-Ala-Ser-Asp-Tyr-Lys-Ser-Ala-His-Lys-Gly-Phe-Lys-Gly-Val-Asp-NH2
MBP 140-154 (SEQ ID NO:4):
   H-Gly-Phe-Lys-Gly-Val-Asp-Ala-Gln-Gly-Thr-Leu-Ser-Lys-Ile-Phe-NH2

The term "peptide" is used in the normal sense to mean a series of residues, typically L-amino acids, connected one to the other, typically by peptide bonds between the α-amino and carboxyl groups of adjacent amino acids. The term includes modified peptides and synthetic peptide analogues.

The peptide of the present invention may be made using chemical methods (Peptide Chemistry, A practical Textbook. Mikos Bodansky, Springer-Verlag, Berlin.). For example, peptides can be synthesized by solid phase techniques (Roberge JY et al (1995) Science 269: 202-204), cleaved from the resin, and purified by preparative high performance liquid chromatography (e.g., Creighton (1983) Proteins Structures And Molecular Principles, WH Freeman and Co, New York NY). Automated synthesis may be achieved, for example, using the ABI 43 1 A Peptide Synthesizer (Perkin Elmer) in accordance with the instructions provided by the manufacturer.

The peptide may alternatively be made by recombinant means, or by cleavage from a longer polypeptide. For example, the peptide may be obtained by cleavage from the myelin basic protein, which may be followed by modification of one or both ends. The composition of a peptide may be confirmed by amino acid analysis or sequencing (e.g., the Edman degradation procedure).

For practical purposes, there are various other characteristics which the peptide may show. For example, it is important that the peptide is sufficiently stable *in vivo* to be therapeutically useful. The half-life of the peptide in vivo may be at least 10 minutes, 30 minutes, 4 hours, or 24 hours.

The peptide may also demonstrate good bioavailability *in vivo.* The peptide may maintain a conformation *in vivo* which enables it to bind to an MHC molecule at the cell surface without due hindrance.

In one aspect the composition may comprise an MBP peptide that has at least 50%, 55%, 60%, 65%, 70%, 75%, 80%, 85%, 90%, 95% or 100% sequence identity to any one of SEQ ID NOs: 1 to 4. In a preferred aspect the peptide has at least 80%, 90%, 95%, 97% or 99% sequence identity to any one of SEQ ID NOs: 1 to 4.

Sequence identity may be assessed by any convenient method. However, for determining the degree of sequence identity between sequences, computer programs that make multiple alignments of sequences are useful, for instance Clustal W (Thompson et al., (1994) Nucleic Acids Res., 22: 4673-4680). Programs that compare and align pairs of sequences, like ALIGN (Myers et al., (1988) CABIOS, 4: 1-17), FASTA (Pearson et al., (1988) PNAS, 85:2444-2448; Pearson (1990), Methods Enzymol., 183: 63-98) and gapped BLAST (Altschul et al., (1997) Nucleic Acids Res., 25: 3389-3402) are also useful for this purpose. Furthermore, the Dali server at the European Bioinformatics institute offers structure-based alignments of protein sequences (Holm (1993) J. Mol. Biol., 233: 123-38; Holm (1995) Trends Biochem. Sci., 20: 478-480; Holm (1998) Nucleic Acid Res., 26: 316-9).

Multiple sequence alignments and percent identity calculations may be determined using the standard BLAST parameters, (using sequences from all organisms available, matrix Blosum 62, gap costs: existence 11, extension 1).

Alternatively, the following program and parameters may be used: Program: Align Plus 4, version 4.10 (Sci Ed Central Clone Manager Professional Suite). DNA comparison: Global comparison, Standard Linear Scoring matrix, Mismatch penalty = 2, Open gap penalty = 4, Extend gap penalty = 1. Amino acid comparison: Global comparison, BLOSUM 62 Scoring matrix.

Thus included in the scope of the invention are variants of the stated or given sequences, as long as the variant retains the functional activity of the parent i.e. the variants are functionally equivalent, in other words they have or exhibit an activity of the parent peptide as defined herein. Such variants may comprise amino acid substitutions, additions or deletions (including truncations at one or both ends) of the parent sequence e.g. of one or more e.g. 1 to 14 amino acids.

A substitution may be a conservative substitution. As used herein, a "conservative substitution" refers to changing amino acid identity at a given position to replace with an amino acid of approximately equivalent size, charge and/or polarity. Examples of natural conservative substitutions of amino acids include the following 8 substitution groups (designated by the conventional one-letter code): (1) M, I, L, V; (2) F, Y, W; (3) K, R, (4) A, G; (5) S, T; (6) Q, N; (7) E, D; and (8) C, S.

Also included are functionally-equivalent derivatives in which one or more of the amino acids are chemically derivatised, e.g. substituted with a chemical group.

The peptides for use according to the invention can comprise portions or fragments of SEQ ID Nos1 to 4, provided that the peptide retains the required activity. Portions or fragments of SEQ ID Nos: 1 to 4 may for example be from 6 to 16 residues in length, e.g. 6, 7, 8, 9, 10, 11, 12, 13, 14, 15 or 16 residues in length.

The peptides for use according to the invention may be comprised within a longer peptide, i.e. a peptide which comprises any of SEQ ID Nos:1-4. The peptides for use according to the present invention may thus comprise between 8 and 30 amino acids, for example 8 to 25 amino acids, 8 to 20 amino acids, 8 to 15 amino acids or 8 to 12 amino acids. In one aspect the peptide of the present invention may thus be 8, 9, 10, 11, 12, 13, 14, 15, 16, 17, 18, 19, 20, 21, 22, 23, 24, 25, 26, 27, 28, 29 or 30 amino acids in length.

A peptide composition for use according to the invention may comprise the amino acid sequences according to the invention as described herein. In one aspect the peptide composition comprises only the amino acid sequences according to the invention as described herein, i.e. it does not comprise additional peptides other than those according to the invention.

The peptides may be formulated into the composition as neutral or salt forms. Pharmaceutically acceptable salts include the acid addition salts (formed with free amino groups of the peptide) and which are formed with inorganic acids such as, for example, hydrochloric or phosphoric acids, or such organic acids such as acetic, oxalic, tartaric and maleic acid. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine and procaine.

### APITOPES

In an adaptive immune response, T lymphocytes are capable of recognising internal epitopes of a protein antigen. APCs take up protein antigens and degrade them into short peptide fragments. A peptide may bind to a major histocompatibility complex (MHC class II) inside the cell and be carried to the cell surface. When presented at the cell surface in conjunction with an MHC molecule, the peptide may be recognised by a T cell (via the T cell receptor (TCR), in which case the peptide is a T cell epitope.

An epitope is thus a peptide derivable from an antigen which is capable of binding to the peptide-binding groove of an MHC molecule and being recognised by a T cell.

The minimal epitope is the shortest fragment derivable from an epitope, which is capable of binding to the peptide-binding grove of an MHC class I or II molecule and being recognised by a T cell. For a given immunogenic region, it is typically possible to generate a "nested set" of overlapping peptides which act as epitopes, all of which contain the minimal epitope but differ in their flanking regions.

By the same token, it is possible to identify the minimal epitope for a particular MHC molecule: T cell combination by measuring the response to truncated peptides. For example, if a response is obtained to the peptide comprising residues 1-15 in the overlapping library, sets which are truncated at both ends (i.e. 1-14, 1-13, 1-12 etc. and 2-15, 3-15, 4-15 etc.) can be used to identify the minimal epitope.

The present inventors have previously determined that there is a link between the capacity of a peptide to bind to an MHC molecule and be presented to a T cell without further processing, and the peptide's capacity to induce antigen-specific immune tolerance *in vivo* (WO 02/16410). If a peptide is too long to bind the peptide binding groove of an MHC molecule without further processing (e.g. trimming), or binds in an inappropriate conformation then it will not be tolerogenic *in vivo.* If, on the other hand, the peptide is of an appropriate size and conformation to bind directly to the MHC peptide binding groove and be presented to a T cell, then this peptide can be predicted to be useful for tolerance induction.

It is thus possible to investigate the tolerogenic capacity of a peptide by investigating whether it can bind to an MHC molecule and be presented to a T cell without further antigen processing *in vitro.*

MBP apitopes (Antigen Processing-Independent epiTOPES) are capable of binding to an MHC molecule and stimulating a response from MBP specific T cells without further antigen processing. Such apitopes can be predicted to cause tolerance to MBP, following the rule-based method described in WO 02/16410.

Peptides that bind to MHC class I molecules are typically 7 to 13, more usually 8 to 10 amino acids in length. The binding of the peptide is stabilised at its two ends by contacts between atoms in the main chain of the peptide and invariant sites in the peptide-binding groove of all MHC class I molecules. There are invariant sites at both ends of the groove which bind the amino and carboxy termini of the peptide. Variations in peptide length are accommodated by a kinking in the peptide backbone, often at proline or glycine residues that allow flexibility.

Peptides which bind to MHC class II molecules are typically between 8 and 20 amino acids in length, more usually between 10 and 17 amino acids in length, and can be longer (for example up to 40 amino acids). These peptides lie in an extended conformation along the MHC II peptide-binding groove which (unlike the MHC class I peptide-binding groove) is open at both ends. The peptide is held in place mainly by main-chain atom contacts with conserved residues that line the peptide-binding groove.

In a preferred embodiment, the peptide derived from MBP is capable of binding to an MHC class II molecule without further processing.

### PORTION

The peptide of the present invention may comprise all or a portion of the MBP derived peptides shown as SEQ ID Nos: 1 to 4.

The term "portion" refers to a peptide that is derived from SEQ ID Nos: 1 to 4 and contains at least a minimal epitope, that is, the peptide is capable of binding to the peptide-binding grove of an MHC class I or II molecule, being recognised by a T cell and inducing tolerance.

### UVEITIS

Clinically, uveitis is commonly classified as one of the following based on the part of the eye which is primarily affected: anterior uveitis, intermediate uveitis, posterior uveitis or panuveitis uveitis.

Anterior uveitis includes iridocyclitis and iritis. Iritis is the inflammation of the anterior chamber and iris, while iridocyclitis includes inflammation in the ciliary body.

Intermediate uveitis (pars planitis) commonly refers to vitritis - inflammation of cells in the vitreous cavity, associated with deposition of inflammatory material on the pars plana. An inflamed vitreous body, or a bleeding or a protein precipitation caused by inflammation is characterised as Intermediate uveitis. In these experiments, the intermediate uveitis symptoms are incorporated in the scoring using the TEFI images. As shown in Example 3, the score 4 (x2) making up to a total of 8 is caused by a white-out. In Figure 4, the TEFI image for the highest score reflects intermediate uveitis. In one aspect of the invention the uveitis is intermediate uveitis.

Posterior uveitis (chorioretinitis) is the inflammation of the retina and choroid regions.

Panuveitis uveitis is a general term referring to inflammation affecting all layers of the uvea.

In one aspect the uveitis is idiopathic uveitis.

In a preferred embodiment of the present invention the uveitis is posterior uveitis. In another aspect of the invention the uveitis is anterior uveitis. In another aspect of the invention the uveitis is intermediate uveitis.

Uveitis can also be classified as either infectious or non-infectious, with uveitis related to autoimmune diseases (i.e. primarily non-infectious) being more common in developed countries. The common animal models used to study uveitis are also driven by autoimmunity, showing a clear association between the two. It is predicted that 25-30% of uveitis is associated with systemic autoimmune or autoinflammatory diseases.

The uveitis according the present invention described herein is preferably non-infectious uveitis, even more preferably autoimmune uveitis.

### TOLERANCE

T cell epitopes play a central role in the adaptive immune response to any antigen, whether self or foreign. The central role played by T cell epitopes in hypersensitivity diseases (which include allergy, autoimmune diseases and transplant rejection) has been demonstrated through the use of experimental models. It is possible to induce inflammatory or allergic diseases by injection of the autoantigen or synthetic peptides (based on the structure of T cell epitopes) in combination with adjuvant.

By contrast, it has been shown to be possible to induce immunogenic tolerance towards particular antigens by administration of peptide epitopes in soluble form. Administration of soluble peptide has been demonstrated as an effective means of inhibiting disease in experimental autoimmune encephalomyelitis (EAE - a model for multiple sclerosis (MS)) (Metzler and Wraith (1993) Int. Immunol. 5:1159-1165; Liu and Wraith (1995) Int. Immunol. 7:1255-1263; Anderton and Wraith (1998) Eur. J. Immunol. 28:1251-1261); and experimental models of arthritis, diabetes, and uveoretinitis (reviewed in Anderton and Wraith (1998) as above). This has also been demonstrated as a means of treating an ongoing disease in EAE (Anderton and Wraith (1998) as above).

Tolerance is the failure to respond to an antigen. Tolerance to self antigens is an essential feature of the immune system, when this is lost, autoimmune disease can result. The adaptive immune system must maintain the capacity to respond to an enormous variety of infectious agents while avoiding autoimmune attack of the self antigens contained within its own tissues. This is controlled to a large extent by the sensitivity of immature T lymphocytes to apoptotic cell death in the thymus (central tolerance). However, not all self antigens are detected in the thymus, so death of self-reactive thymocytes remains incomplete. There are thus also mechanisms by which tolerance may be acquired by mature self-reactive T lymphocytes in the peripheral tissues (peripheral tolerance). A review of the mechanisms of central and peripheral tolerance is given in Anderton et al (1999) Immunological Reviews 169:123-137.

Available data suggests that uveitis can result from reactive T cells generated from retinal proteins, driving inflammation and mirroring the effect of a chronic infection. The composition according to the present invention is capable of inducing tolerance to self-antigens such as MBP, such that when administered to a subject, it may reinstate tolerance to the MBP protein and curtail the pathogenic immune response.

### COMPOSITION

The composition according to the present invention may be for prophylactic or therapeutic use in patients with uveitis.

When administered for prophylactic use, the composition may reduce or prevent the generation of an immune response to MBP. The level of immune response is less than would be obtained if the patient had not been treated with the composition. The term "reduce" indicates that a partial reduction in immune response is observed, such as a 50%, 70%, 80% or 90% reduction in the response that would have been observed if the patient had not been treated with the composition (or in the response observed in an untreated patient over the same time-period). The term "prevent" indicates that no appreciable immune response to MBP is observed.

When administered for therapeutic use, the composition may suppress an already on-going immune response to MBP. The term "suppress" indicates a reduction in the level of an on-going immune response, compared to the level before peptide treatment, or the levels which would have been observed at the same time point had the treatment not been given.

Treatment with the composition according to the present invention may cause a reduction in level of any or all of the following:
i) MBP autoantibodies
ii) proinflammatory CD4+ T cells specific for MBP
iii) B cells secreting MBP autoantibodies.

Detection of all of the factors can be carried out by techniques known in the art, such as ELISA, flow cytometry etc.

Treatment with the composition of the present invention may also or alternatively cause anergy in CD4+ T cells specific for MBP. Anergy can be detected by, for example, subsequent challenge with MBP *in vitro.* Treatment with the composition of the present invention may cause generation of antigen-specific regulatory T cells, for example characterised by transcription factors c-Maf and NFIL3, and negative co-stimulatory molecules LAG-3, TIGIT, PD-1 and TIM-3 (see Burton et al. Nature Communications (2014) Article number 4741).

### FORMULATION

The composition according to the invention as described herein may be prepared as an injectable, either as liquid solution or suspension; solid form suitable for solution in, or suspension in, liquid prior to injection may also be prepared. The preparation may also be emulsified, or the peptides encapsulated in liposomes. The peptide may alternatively be encapsulated in a carrier or bound to the surface of a carrier, for example a nanoparticle. The active ingredients may be mixed with excipients which are pharmaceutically acceptable and compatible with the active ingredient. Suitable excipients are, for example, water, saline (for example, phosphate-buffered saline), dextrose, glycerol, ethanol, or the like and combinations thereof.

In addition, if desired, the composition may contain minor amounts of auxiliary substances such as wetting or emulsifying agents and/or pH buffering agents. Buffering salts include phosphate, citrate, acetate. Hydrochloric acid and/or sodium hydroxide may be used for pH adjustment. For stabilisation, disaccharides may be used such as sucrose or trehalose.

After formulation, the composition may be incorporated into a sterile container which is then sealed and stored at a low temperature, for example 4°C, or it may be freeze-dried.

Conveniently the composition is prepared as a lyophilised (freeze-dried) powder. Lyophilisation permits long-term storage in a stabilised form. Lyophilisation procedures are well known in the art, see for example http://www.devicelink.com/ivdt/archive/97/01/006.html. Bulking agents are commonly used prior to freeze-drying, such as mannitol, dextran or glycine.

The composition may be administered in a convenient manner such as by the oral, intravenous (where water soluble), intramuscular, subcutaneous, sublingual, intranasal, intradermal or suppository routes or implanting (e.g. using slow release molecules or devices).

The composition may advantageously be administered via intranasal, subcutaneous or intradermal routes. In a preferred embodiment administration is intradermal.

The pharmaceutical composition for use according to the invention may be used to treat a human subject. Typically, a physician will determine the actual dosage which will be most suitable for an individual subject and it will vary with the age, weight and response of the particular patient.

The composition as described herein is typically administered in an "effective amount"; that is, an amount effective to elicit any one or more inter alia of a therapeutic or prophylactic effect. Persons skilled in the art would be able, by routine experimentation, to determine an effective, non-toxic amount to include in a pharmaceutical composition or to be administered for the desired outcome. In general, composition as disclosed herein can be administered in a manner compatible with the route of administration and physical characteristics of the recipient (including health status) and in such a way that it elicits the desired effect(s) (i.e. therapeutically effective and/or protective). For example, the appropriate dosage of a composition may depend on a variety of factors including, but not limited to, a subject's physical characteristics (e.g., age, weight, sex), and other factors that may be recognized by persons skilled in the art. Other illustrative examples of general considerations that may be considered when determining, for example, an appropriate dosage of the compositions are discussed by Gennaro (2000, "Remington: The Science and Practice of Pharmacy", 20th edition, Lippincott, Williams, & Wilkins; and Gilman et al., (Eds), (1990), "Goodman And Gilman's: The Pharmacological Bases of Therapeutics", Pergamon Press).

It is expected that the amount will fall in a relatively broad range that can be determined through methods known to persons skilled in the art, having regard to some of the considerations outlined above.

The peptide and composition of the invention may be used to treat a human subject. The subject according to the invention preferably has posterior uveitis. The subject may have T cells specific to MBP.

In a preferred embodiment a "dose escalation" protocol may be followed, where a plurality of doses is given to the patient in ascending concentrations. Such an approach has been used, for example, for phospholipase A2 peptides in immunotherapeutic applications against bee venom allergy (Müller et al (1998) J. Allergy Clin Immunol. 101:747-754 and Akdis et al (1998) J. Clin. Invest. 102:98-106).

### KIT

Peptides derived from MBP may be administered together, in the form of a mixed composition or cocktail. However, there may be circumstances in which it is preferable to provide the peptides separately in the form of a kit, for simultaneous, separate, sequential or combined administration.

For example, the kit may comprise the three peptides in separate containers. The contents of the containers may or may not be combined prior to administration.

The kit may also comprise mixing and/or administration means (for example a vapouriser for intranasal administration; or a syringe and needle or other medical device for subcutaneous/intradermal dosing). The kit may also comprise instructions for use.

The composition/kit may be used to suppress or prevent the production of MBP autoantibodies *in vivo.* In particular, the composition/kit may be used to treat and/or prevent uveitis in a subject.

### ANIMAL MODEL

An animal model of uveitis is described herein. As discussed in the Examples, the animal model provides the advantage that uveitis is induced without induction of encephalomyelitis (EAE).

Thus described herein is an animal model for uveitis, wherein the animal comprises increased levels of myelin basic protein (MBP) and interphotoreceptor retinoid binding protein (IRBP) compared to a control animal.

The term "increased levels of MBP and IRBP" is intended to encompass an increased level of any of the full length MBP or IRBP sequence, for example MBP or IRBP peptides.

The MBP may be from, or derived from, human or guinea pig (gpMBP). The sequences of human and guinea pig MBP are set out below:

### Human MBP (UniProtKB entry P02686 - full length human MBP sequence)

MGNHAGKRELNAEKASTNSETNRGESEKKRNLGELSRTTSEDNEVFGEADANQNNGTSSQ
DTAVTDSKRTADPKNAWQDAHPADPGSRPHLIRLFSRDAPGREDNTFKDRPSESDELQTI
QEDSAATSESLDVMASQKRPSQRHGSKYLATASTMDHARHGFLPRHRDTGILDSIGRFFG
GDRGAPKRGSGKDSHHPARTAHYGSLPQKSHGRTQDENPVVHFFKNIVTPRTPPPSQGKG
RGLSLSRFSWGAEGQRPGFGYGGRASDYKSAHKGFKGVDAQGTLSKIFKLGGRDSRSGSP
MARR (SEQ ID NO: 5)

Other isoforms of MBP are also known, for example UniProtKB P02686-3, P02686-4, P02686-5 and P02686-6.

### Guinea pig MBP (UniProtKB entry P25188)

ASQKRPSQRHGSKYLATASTMDHARHGFLPRHRDTGILDSIGRFFGSDRAAPKRGSGKDS
HHAARTTHYGSLPQKSQRSQDENPVVHFFKNIVTPRTPPPSQGKGRGLSLSRFSWGAEGQ
KPGFGYGGRADYKSKGFKGAHDAQGTLSKIFKLGGRDSRSGSPMARR (SEQ ID NO: 6)

The IRBP may be human IRBP, or derived from human IRBP. The sequence of human IRBP is set out below (UniProtKB entry P10745):





















 (SEQ ID NO: 7)

The IRBP may be bovine IRBP (SEQ ID NO: 8) (UniProtKB entry P12661):

MVRKWALLLP MLLCGLTGPA HLFQPSLVLE MAQVLLDNYC FPENLMGMQG
60 70 80 90 100
AIEQAIKSQE ILSISDPQTL AHVLTAGVQS SLNDPRLVIS YEPSTLEAPP
110 120 130 140 150
RAPAVTNLTL EEIIAGLQDG LRHEILEGNV GYLRVDDIPG QEVMSKLRSF
160 170 180 190 200
LVANVWRKLV NTSALVLDLR HCTGGHVSGI PYVISYLHPG STVSHVDTVY
210 220 230 240 250
DRPSNTTTEI WTLPEALGEK YSADKDVVVL TSSRTGGVAE DIAYILKQMR
260 270 280 290 300
RAIVVGERTV GGALNLQKLR VGQSDFFLTV PVSRSLGPLG EGSQTWEGSG
310 320 330 340 350
VLPCVGTPAE QALEKALAVL MLRRALPGVI QRLQEALREY YTLVDRVPAL
360 370 380 390 400
LSHLAAMDLS SVVSEDDLVT KLNAGLQAVS EDPRLQVQVV RPKEASSGPE
410 420 430 440 450
EEAEEPPEAV PEVPEDEAVR RALVDSVFQV SVLPGNVGYL RFDSFADASV
460 470 480 490 500
LEVLGPYILH QVWEPLQDTE HLIMDLRQNP GGPSSAVPLL LSYFQSPDAS
510 520 530 540 550
PVRLFSTYDR RTNITREHFS QTELLGRPYG TQRGVYLLTS HRTATAAEEL

560 570 580 590 600
AFLMQSLGWA TLVGEITAGS LLHTHTVSLL ETPEGGLALT VPVLTFIDNH
610 620 630 640 650
GECWLGGGVV PDAIVLAEEA LDRAQEVLEF HRSLGELVEG TGRLLEAHYA
660 670 680 690 700
RPEVVGQMGA LLRAKLAQGA YRTAVDLESL ASQLTADLQE MSGDHRLLVF
710 720 730 740 750
HSPGEMVAEE APPPPPVVPS PEELSYLIEA LFKTEVLPGQ LGYLRFDAMA
760 770 780 790 800
ELETVKAVGP QLVQLVWQKL VDTAALVVDL RYNPGSYSTA VPLLCSYFFE
810 820 830 840 850
AEPRRHLYSV FDRATSRVTE VWTLPHVTGQ RYGSHKDLYV LVSHTSGSAA
860 870 880 890 900
EAFAHTMQDL QRATIIGEPT AGGALSVGIY QVGSSALYAS MPTQMAMSAS
910 920 930 940 950
TGEAWDLAGV EPDITVPMSV ALSTARDIVT LRAKVPTVLQ TAGKLVADNY
960 970 980 990 1000
ASPELGVKMA AELSGLQSRY ARVTSEAALA ELLQADLQVL SGDPHLKTAH
1010 1020 1030 1040 1050
IPEDAKDRIP GIVPMQIPSP EVFEDLIKFS FHTNVLEGNV GYLRFDMFGD
1060 1070 1080 1090 1100
CELLTQVSEL LVEHVWKKIV HTDALIVDMR FNIGGPTSSI SALCSYFFDE
1110 1120 1130 1140 1150
GPPILLDKIY NRPNNSVSEL WTLSQLEGER YGSKKSMVIL TSTLTAGAAE
1160 1170 1180 1190 1200
EFTYIMKRLG RALVIGEVTS GGCQPPQTYH VDDTDLYLTI PTARSVGAAD
1210 1220 1230 1240 1250
GSSWEGVGVV PDVAVPAEAA LTRAQEMLQH TPLRARRSPR LHGRRKGHHR
1260 1270 1280
QSQGRAGSLG RNQGVVRPEV LTEAPSGQKR GLLQCG

The IRBP may be a peptide derived from IRBP, preferably IRBP1177-1191 ADGSSWEGVGVVPDV (SEQ ID NO: 9).

The level of MBP and IRBP is increased over a suitable control animal, for example an animal to which exogenous or transgenic MBP and IRBP has not been administered.

The level of MBP and IRBP may be increased in the animal by any suitable means. For example, this may be by immunising the animal with MBP and IRBP, as described in the present Examples.

Alternatively, said increase may be achieved by administering one or more vectors comprising one or more nucleic acid molecules which encode MBP and/or IRBP. The vector may be a viral vector.

Described herein is a method for producing an animal model of uveitis, said method comprising increasing the level of MBP and IRBP in an animal.

The method may comprise increasing the level of MBP and IRBP by administering MBP and IRBP to the animal, that is by immunising the animal with MBP and IRBP e.g. as described in the present Examples.

The animal model has features which are relevant to uveitis. For example, in the animal model, clinical signs of uveitis may be increased compared to equivalent control animals in which the levels of MBP and IRBP are not increased.

The MBP and/or IRBP may be human MBP and/or IRBP.

The MBP or IRBP may be a part or fragment or portion of MBP or IRBP, provided that the required function is retained in the animal model. The IRBP may be the IRBP1177-1191 peptide (ADGSSWEGVGWPDV (SEQ ID NO: 9) - also referred to as R16).

The MBP and/or IRBP may be administered to the animal in any suitable manner, for example oral, intravenous (where water soluble), intramuscular, subcutaneous, sublingual, intranasal, intradermal or suppository routes or implanting (e.g. using slow release molecules).

Administration may be sub-cutaneous.

A suitable dose of the MBP that is administered to the animal may be about 10, 20, 30, 40, 50, 60, 70, 80, 90, 100, 110, 120, 130, 140, 150, 160, 170, 180, 190, 200, 300, 400 or 500µg. In one aspect about 100 µg of MBP is administered.

A suitable dose of the IRBP that is administered to the animal may be about 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90 or 100µg. In one aspect about 20 µg of IRBP is administered.

The MBP and/or IRBP may be administered with an adjuvant, for example Freund's adjuvant.

A nucleic acid encoding MBP and/or IRBP may be provided using methods which are well known in the art, for example based on the MBP and/or IRBP sequences described herein.

The nucleic acid may be natural, synthetic or recombinant. It may be double or single stranded, it may be DNA or RNA or combinations thereof. It may, for example, be cDNA, a PCR product, genomic sequence or mRNA.

The nucleotide sequence may be codon optimised for production in the host/host cell of choice.

Delivery of the nucleotide sequence encoding MBP and/or IRBP may be mediated by viral infection. Suitable viral vectors are well known in the art. For example the viral vector may be an adenovirus, retrovirus or lentivirus.

Production of the animal model may comprise multiple administrations of the vector.

The vector, for example adenovirus, may be administered at 3 week intervals, For example, the vector may be administered at an interval of 18-25, 18-23, 19-23 or 20-22 days. The vector may be administered at an interval of 20, 21 or 22 days.

The animal model may comprise administering the adenovirus vector on at least two occasions. For example the adenovirus vector may be administered on two or three occasions.

The vector, for example adenovirus, may be administered at a dose of 10⁸ to 10¹¹ viral particles per administration. In particular, the adenovirus may be administered at a dose of 10⁹ to 10¹¹ viral particles per administration. The adenovirus may be administered at a dose of 10⁹ viral particles per administration.

The vector may be administered by any suitable means. For example, in the case of an adenoviral vector, the vector may be administered by intramuscular injection.

The animal may be a mammal. For example the animal may be a mouse, rat, rabbit, guinea pig or primate. Preferably, the animal is a rat.

The animal may be a Lewis rat, preferably a female Lewis rat.

By way of example, the model described in the present Examples was generated by administration by subcutaneous injection with 200 microliters of an emulsion containing 20 micrograms of IRBP1177-1191 and 100 micrograms of human (huMBP) or guinea pig Myelin Basic Protein (gpMBP) in incomplete Freund's adjuvant supplemented with 5 mg/ml Mycobacterium Tuberculosis H37Ra. As discussed herein, the model provides the advantage that it induces uveitis with marginal induction of encephalomyelitis.

The invention will now be described with reference to the following Examples.

### EXAMPLES

### Example 1

This study aimed at studying the effect, in Lewis rats, of immunisations with guinea pig myelin basic protein (gpMBP), human myelin basic protein (huMBP) and/or interphotoreceptor retinoid-binding protein with regard to development of encephalomyelitis (EAE) and/or uveitis (EAU).

### Experimental outline

Adult female Lewis rats were randomly allocated to experimental groups and allowed to acclimatise for one week. On Day 0, animals were administered by subcutaneous injection with 200 microliters of an emulsion containing 20 micrograms of IRBP1177-1191 (Cambridge Bioscience, UK) and/or 100 micrograms of human (Fitzgerald Industries International, MA, US) or guinea pig Myelin Basic Protein (gpMBP) in incomplete Freund's adjuvant (Sigma, UK) supplemented with 5 mg/ml Mycobacterium Tuberculosis H37Ra (500 µg per rat) (Becton Dickenson, UK). From Day 0 until the end of the experiment, animals were weighed daily. From Day 0 until the end of the experiment, animals were scored daily for clinical signs of experimental autoimmune encephalomyelitis to include limpness, paresis and/or paralysis of the tail, hind limbs and/or front limbs. From Day 0 until the end of the experiment, animals were scored twice weekly for clinical signs of uveitis. On Day 21 (Groups 1, 2 and 3) or Day 28 (Groups 4 and 5), animals were culled and the following samples were dissected out and stored in tissue fixative for optional histopathology analysis: brain, spinal cord, right and left eyes.

### Treatment Groups and Dosages

Groups 1, 2 and 3 are n=8 per group
Groups 4 and 5 are n=14 per group

**n/a: not applicable, IRBP: interphotoreceptor binding protein, gpMBP: guinea pig myelin basic protein, huMBP: human myelin basic protein, CFA: complete Freund's adjuvant, SC: subcutaneous injection, *500 µg per rat**

| | **Group** | **Antigenic Challenge** | **Necropsy** |
|---|---|---|---|
| 1 | IRBP | Day 0: 200 µl emulsion containing 20 µg IRBP in CFA 5 mg/ml *Mycobacterium Tuberculosis** | Day 21 |
| 2 | gpMBP | Day 0: 200 µl emulsion containing 100 µg gpMBP in CFA 5 mg/ml *Mycobacterium Tuberculosis** | |
| 3 | IRBP & gpMBP | Day 0: 200 µl emulsion containing 20 µg IRBP and 100 µg gpMBP in CFA 5 mg/ml *Mycobacterium Tuberculosis** | |
| 4 | huMBP | Day 0: 200 µl emulsion containing 100 µg huMBP in CFA 5 mg/ml *Mycobacterium Tuberculosis** | Day 28 |
| 5 | IRBP & huMBP | Day 0: 200 µl emulsion containing 20 µg IRBP and 100 µg huMBP in CFA 5 mg/ml *Mycobacterium Tuberculosis** | |

### Readouts

### Non-specific clinical observations

From Day 0 until the end of the experiment, animals were checked daily for non-specific clinical signs to include abnormal posture (hunched), abnormal coat condition (piloerection) and abnormal activity levels (reduced or increased activity).

### Bodyweights

From Day 0 until the end of the experiment, animals were weighed daily.

### Clinical Scores (Uveitis)

From Day 0 until the end of the experiment, animals were visually scored twice per week for clinical signs of anterior uveitis using the following scoring system. The maximum possible score was 3. Data were graphed (Mean ± SEM).

| | **Description** |
|---|---|
| 0 | Normal |
| 1 | Slight iris vessel dilation and thickened iris stroma, a few scattered inflammatory cells |
| 2 | Engorged blood vessels in the iris, abnormal pupil contraction |
| 3 | Hazy anterior chamber |

From Day 0 until the end of the experiment, animals were scored twice per week for clinical signs of intermediate and posterior uveitis. Retinal images were captured using TEFI in non-anaesthetised but restrained animals following pupil dilatation using Tropicamide 1% then Phenylephrine hydrochloride 2.5%. Retinal images were scored using the following system. The maximum possible score was 6. Data were graphed (Mean ± SEM). TEFI images displays symptoms of both intermediate and posterior uveitis.

| | **Description** |
|---|---|
| | **Retinal vessels** |
| 1 | 1 vessel engorged |
| 2 | More than 1 vessel engorged |
| 3 | All main vessels engorged |

| | **Retinal tissue Infiltration** |
|---|---|
| 1 | 1-2 small areas of retinal damage |
| 2 | 3+ small areas or 1 + large area |
| 3 | Not visible (white-out or severe detachment) (caused by e.g.vitreous body inflammation or bleeding |

### Results

### Non-specific clinical observations

From Day 0 until the end of the experiment, animals did not show any non-specific clinical signs such as abnormal posture (hunched), abnormal coat condition (piloerection) or abnormal activity levels (reduced or increased activity). In animals administered with gpMBP, a significant bodyweight loss was observed from Day 11 until the end of the experiment on Day 21 when compared to the IRBP-administered group (p < 0.0001).

In animals administered with IRBP and gpMBP, a significant bodyweight loss was observed from Day 11 until the end of the experiment on Day 21 when compared to the IRBP-administered group (p < 0.05 on Day 11, p < 0.001 on Day 12, p < 0.0001 from Day 13 until Day 20 and p < 0.001 on Day 21). There was no difference in bodyweight loss between animals administered with gpMBP alone and animals administered with gpMBP and IRBP. There was no significant bodyweight loss observed in animals administered with IRBP alone, human MBP or human MBP with IRBP.

### Clinical scores - Uveitis

Animals with an average score for both eyes greater than 0.5 on at least two consecutive TEFI sessions (see Figure 2) or a score greater than 1.0 on any occasion were considered to have developed clinical signs of posterior uveitis. Based on this criterion, the incidence of EAU was:

| **Group** | **Incidence** | **Onset (Day)** | **Maximum Score** |
|---|---|---|---|
| IRBP | 100% (8/8) | 10.5 ± 0.5 | 5.6 ± 0.3 |
| gpMBP | 50% (4/8) | 11.5 ± 0.5 | 1.1 ± 0.2 |
| gpMBP and IRBP | 100% (8/8) | 9.0 ± 0.8 | 4.9 ± 0.4 |
| huMBP | 100% (14/14) | 10.2 ± 0.7 | 4.1 ± 0.5 |
| huMBP and IRBP | 100% (14/14) | 9.9 ± 0.5 | 5.0 ± 0.3 |

Due to the lack of pigmentation in the retina of Lewis rats, the scoring system below was used. This is due to the inability to identify areas of infiltration causing haziness and cuffing on a pale background. Inflammation of the optic disc could not be clearly identified and was not therefore scored, also, no structural damage was observed. Infiltration of retinal vessels could be identified by engorged appearance on a scale of 0 - 3, mild to severe. Retinal inflammation was identified by the appearance of small to large areas of redness caused by increase in vascularisation also on a scale of 0 - 3, giving a total possible score of 6.

| | **Description** |
|---|---|
| | **Retinal vessels** |
| 1 | 1 vessel engorged |
| 2 | More than 1 vessel engorged |
| 3 | All main vessels engorged |

| | **Retinal tissue Infiltration** |
|---|---|
| 1 | 1-2 small areas of retinal damage |
| 2 | 3+ small areas or 1 + large area |
| 3 | Not visible (white-out or severe detachment) |

The incidence of clinical signs of posterior uveitis was maximal in all experimental groups administered with IRBP, either alone or in combination with gpMBP or huMBP. Clinical signs of EAU were also observed in 50% of the gpMBP-administered animals and in 100% of the huMBP-administered animals in absence of IRBP (see Figure 1).

The onset of clinical signs of EAU did not differ statistically between experimental groups.

The severity of the clinical signs of EAU was significantly lower in the gpMBP-administered animals when compared to the experimental group administered with IRBP (p < 0.001), IRBP and gpMBP (p < 0.01), IRBP and huMBP (p < 0.01) or huMBP (p < 0.05).

Clinical signs of anterior uveitis were also observed during the experiment.

Data collected until Day 21 were analysed by two-way ANOVA followed by Dunnett's post-test for multiple comparisons between experimental groups.

In IRBP-administered animals, clinical signs were observed from Day 11. Signs were significantly higher than in the gpMBP-administered group on Day 11, Day 14 and Day 18 (p < 0.0001) (see Figure 3).

In IRBP & gpMBP-administered animals, signs of anterior uveitis were also observed from Day 11 (p < 0.0001 on Day 11, p < 0.01 on Day 14 and Day 18 and p < 0.05 on Day 21) when compared to the gpMBP-administered group.

In IRBP & huMBP-administered animals, signs of anterior uveitis were observed on Day 11 and Day 14 (p < 0.0001) when compared to the gpMBP-administered group.

In huMBP-administered animals, signs of anterior uveitis were observed on Day 14 (p < 0.0001) and Day 18 (p < 0.01) when compared to the gp-MBP administered group.

Guinea pig MBP may induce Experimental autoimmune encephalomyelitis (EAE) in Lewis rats, a model for multiple sclerosis (MS). In this experiment, severe EAE was developed in Lewis rats immunised with gpMBP alone (incidence 8/8 rats; mean max score 3.5) or in combination with IRBP peptide (incidence 8/8 rats; mean max score 3.9) using the scoring system for paralytic symptoms: 0) no abnormality, (0.5) partial tail limpness, (1) complete tail limpness, (2) one hind limb paresis, (2.5) two hind limbs paresis, (3) one hind limb paralysis, (4) two hind limb paralysis, (5) incontinence and (6) moribund. With difference to the gpMBP immunised rats, very mild symptoms were displayed in the other groups: Immunised with IRBP alone (incidence 2/8, mean max score 1.0); huMBP immunised (incidence 3/14, mean max score 1.0) and huMBP in combination with IRBP peptide (incidence 3/14, mean max score 1.0).

### Conclusion

Administration of huMBP resulted in signs of both anterior and posterior uveitis.

When administered at the same time as IRBP clinical signs of both anterior and intermediate/posterior uveitis were increased and provided a robust model for testing for efficacy against autoimmune uveitis.

With addition of the very low incidence and severity of EAE developed after immunisation with human MBP it was decided to use the model of uveitis involving immunisation with human MBP alone or with the addition of the IRBP peptide for studying the effects of the MBP peptides according to the present invention, as described in Example 2.

### Example 2

This example details studies of the effect of ATX-UV-3 in a Lewis rat model of human myelin basic protein (huMBP) and interphotoreceptor retinoid-binding protein (IRBP)-induced uveitis, as described in Example 1.

ATX-UV-3 comprises:
MBP 30-44 (SEQ ID NO:1);
MBP 83-99 (SEQ ID NO:2);
MBP 131-145 (SEQ ID NO:3); and
MBP 140-154 (SEQ ID NO:4)

### Methods

### Experimental outline

Adult female Lewis rats were randomly allocated to experimental groups and allowed to acclimatise for one week. On Day 0, animals were administered by subcutaneous injection with 200 microliters of an emulsion containing 100 micrograms of human Myelin Basic Protein (MBP) (Fitzgerald Industries International, MA, US) in incomplete Freund's adjuvant (Sigma, UK) supplemented with 5 mg/ml Mycobacterium Tuberculosis H37Ra (500 µg per rat)( Becton Dickenson, UK) and 20 micrograms of IRBP 1177-1191 (Cambridge Bioscience, UK). From Day 0 until the end of the experiment, animals were scored twice weekly for clinical signs of uveitis.

On Day 21, clinical scores were analysed and the study was extended to Day 35. On Day 35, animals were culled and eyes (right and left) were dissected out and stored in tissue fixative for histopathology analysis.

**Table 1: Treatment Groups and Dosages**

| (All groups are n=12) | | | | | |
|---|---|---|---|---|---|
| | **Group** | **Treatment** | | | **Disease Induction** |
| | | **Dose** | **Route** | **Regimen** | |
| 1 | Vehicle | n/a | SC | Days -8, -6 and -4 | Day 0: 100 µg huMBP and 20 µg IRBP |
| 2 | ATX -UV-3 | 100 µg | SC | Days -8, -6 and -4 | |

| | | | | | |
|---|---|---|---|---|---|
| n/a: not applicable, IRBP: interphotoreceptor binding protein, huMBP: human myelin basic protein, SC: subcutaneous injection. | | | | | |

### Non-specific clinical observations

From Day 0 until the end of the experiment, animals were checked daily for non-specific clinical signs to include abnormal posture (hunched), abnormal coat condition (piloerection) and abnormal activity levels (reduced or increased activity).

### Clinical Scores (Uveitis)

From Day 0 until the end of the experiment, animals were scored twice per week for clinical signs of ocular inflammation (anterior uveitis) using the following scoring system: (0) normal, (1) slight iris vessel dilation and thickened iris stroma, a few scattered inflammatory cells, or both, (2) engorged blood vessels in the iris, abnormal pupil contraction and (3) hazy anterior chamber. Data were graphed (Mean ± SEM).

**Table 2: Anterior uveitis scoring:**

| **Score** | **Description** |
|---|---|
| 0 | Normal |
| 1 | Slight iris vessel dilation and thickened iris stroma, a few |
| | scattered inflammatory cells |
| 2 | Engorged blood vessels in the iris, abnormal pupil contraction |
| 3 | Hazy anterior chamber |

From Day 0 until the end of the experiment, animals were scored twice per week for clinical signs of uveitis. Retinal images were captured using TEFI in non-anaesthetised but restrained animals following pupil dilatation using Tropicamide 1% then Phenylephrine hydrochloride 2.5%. Retinal images were scored using the modified scoring system described below with a maximum possible score of 10. Data were graphed (Mean ± SEM).

**Table 3: Intermediate/posterior uveitis scoring:**

| **Score** | **Criteria** |
|---|---|
| | **Retinal vessels** |
| 1 | 1 vessel engorged |
| 2 | More than 1 vessel engorged |
| 3 | All main vessels engorged |
| 4 | Not visible due to 'white-out¹' |

| | **Retinal tissue Infiltration** |
|---|---|
| 1 | 1-2 small areas of retinal damage |
| 2 | 3+ small areas or 1 + large area |
| 3 | Widespread damage |
| 4 | Not visible (white-out or severe detachment) |

| | **Structural damage** |
|---|---|
| 1 | Retinal detachment with folding |
| 2 | Not visible (white-out or severe detachment) |

| | |
|---|---|
| ¹ also refers to intermediate uveitis | |

### Results

### Non-specific clinical observations

From Day 0 until the end of the experiment, animals did not show any non-specific clinical signs such as abnormal posture (hunched), abnormal coat condition (piloerection) or abnormal activity levels (reduced or increased activity).

### Clinical scores (Uveitis)

### Posterior uveitis

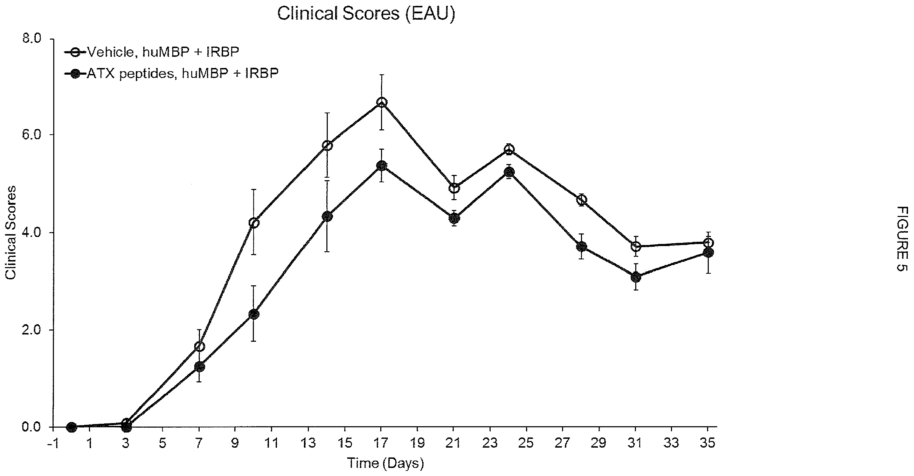

Animals with an average score for both eyes greater than 0.5 on at least two consecutive TEFI sessions or a score greater than 1.0 on any occasion were considered to have developed clinical signs of posterior uveitis (Figure 5).

**Table 4: Incidence of posterior/intermediate EAU:**

| **Group** | **Incidence** | **Onset (Day)** | **Maximum Score** |
|---|---|---|---|
| huMBP/IRBP, Vehicle | 100% (12/12) | 8.3 ± 0.4 | 7.3 ± 0.7 |
| huMBP/IRBP, ATX-UV-3 | 100% (12/12) | 9.8 ± 1.0 | 6.0 ± 0.5 |

Clinical scores were analysed by two-way ANOVA followed by Tukey's post-test for multiple comparisons between experimental groups on each experimental day (Figure 5).

In the huMBP and IRBP-challenged groups, ATX peptide significantly reduced the clinical scores when compared to the vehicle-treated group on Day 10 (p < 0.0001), Day 14 (p < 0.01) and Day 17 (p < 0.05).

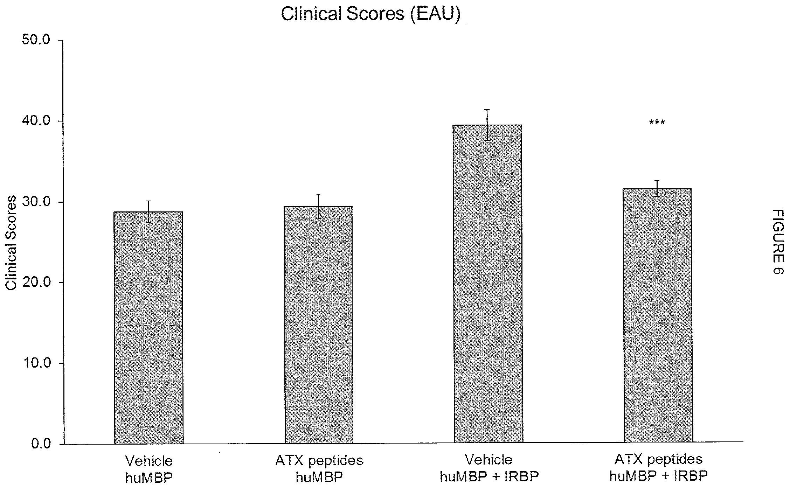

Clinical scores were further analysed by calculating Area Under the Curve (AUC) values and comparing AUC for each experimental group by one-way ANOVA followed by Sidak's post-test for multiple comparisons between all experimental groups (Figure 6).

In the huMBP and IRBP-challenged groups, ATX peptide significantly reduced the clinical scores when compared to the vehicle-treated group (p < 0.001).

### Example 3

A further study was carried out to investigate the efficacy of ATX-UV-3 in an animal model of uveitis. Cyclosporin A was included as a positive control. (A high dose of Cyclosporin A was used which would not be a physiological dose for humans but was chosen for the purpose of validating the model.)

### Experimental outline

Adult female Lewis rats were randomly allocated to experimental groups and allowed to acclimatise for one week. Treatments were administered according to the schedule below. On Day 0, animals were administered by subcutaneous injection with 200 microliters of an emulsion containing 100 micrograms of human Myelin Basic Protein (MBP) in incomplete Freund's adjuvant supplemented with 5 mg/ml Mycobacterium Tuberculosis H37Ra (500 µg per rat) and/or 20 micrograms of IRBP1177-1191. From Day 0 until the end of the experiment, animals were scored twice weekly for clinical signs of anterior and posterior uveitis. On Day 28, animals were culled and eyes (right and left) were dissected out, transferred in tissue fixative then processed for paraffin embedding and stored in paraffin blocks for optional histopathology analysis.

### Treatment groups and dosages

All experimental groups were n=12

The administration volume was 5 ml/kg for PO administration and 100 µl fixed volume per rat for SC administration.

**Table 5**

| | **Group** | **Treatment** | | | **Disease Induction** |
|---|---|---|---|---|---|
| | | **Dose** | **Route** | **Regimen** | |
| 1 | Vehicle | n/a | SC | 13 doses* | Day 0: 100 µg |
| 2 | Cyclosporin A | 20 mg/kg | PO | SID, Day 0-Day 27 | huMBP |
| 3 | ATX peptides | 0.1-100 µg | SC | 13 doses* | |
| 4 | Vehicle | n/a | SC | 13 doses* | Day 0: 100 µg huMBP and 20 µg IRBP |
| 5 | Cyclosporin A | 20 mg/kg | PO | SID, Day 0-Day 27 | |
| 6 | ATX peptides | 0.1-100 µg | SC | 13 doses* | |

| | | | | | |
|---|---|---|---|---|---|
| n/a: not applicable, IRBP, interphotoreceptor binding protein; huMBP, human myelin basic protein; CFA, complete Freund's adjuvant; SC, subcutaneous injection; PO, *per* os, oral gavage; SID, *semel in die,* once daily *ATX peptides or vehicle were administered on the following days and at the following doses: 0.1 µg/rat on Day -22, 1 µg/rat on Day -20, 10 µg/rat on Day -18, 100 µg/rat on Days -15, -13, -11, -8, -6, -4, 3, 10, 17 and 24. | | | | | |

### Readouts

### Non-specific clinical observations

From Day 0 until the end of the experiment, animals were checked daily for non-specific clinical signs to include abnormal posture (hunched), abnormal coat condition (piloerection) and abnormal activity levels (reduced or increased activity). Animals were culled prior to the scheduled end of the study if non-specific clinical signs were judged too severe.

### Bodyweights

From Day 0 until the end of the experiment, animals were weighed daily.

### Clinical scores (Uveitis)

From Day 0 until the end of the experiment, animals were scored twice per week by a single operator blind to treatment for clinical signs of ocular inflammation (anterior uveitis) using the following scoring system: (0) normal, (1) slight iris vessel dilation and thickened iris stroma, a few scattered inflammatory cells, or both, (2) engorged blood vessels in the iris, abnormal pupil contraction and (3) hazy anterior chamber. Data were graphed (Mean ± SEM).

**Table 6**

| **Anterior uveitis scoring:** | |
|---|---|
| | **Description** |
| 0 | Normal |
| 1 | Slight iris vessel dilation and thickened iris stroma, a few scattered inflammatory cells |
| 2 | Engorged blood vessels in the iris, abnormal pupil contraction |
| 3 | Hazy anterior chamber |

From Day 0 until the end of the experiment, animals were scored twice per week by a single operator blind to treatment for clinical signs of posterior uveitis. Retinal images were captured using TEFI in non-anaesthetised but restrained animals following pupil dilatation using Tropicamide 1% then Phenylephrine hydrochloride 2.5%. Retinal images were scored using the modified scoring system described below with a maximum possible score of 10. Data were graphed (Mean ± SEM).

**Table 7**

| **Posterior/intermediate uveitis scoring:** | |
|---|---|
| | **Criteria** |
| | **Retinal vessels** |
| 1 | 1 vessel engorged |
| 2 | More than 1 vessel engorged |
| 3 | All main vessels engorged |
| 4 | Not visible due to 'white-out' |

| | **Retinal tissue Infiltration** |
|---|---|
| 1 | 1-2 small areas of retinal damage |
| 2 | 3+ small areas or 1 + large area |

| | |
|---|---|
| 3 | Widespread damage |
| 4 | Not visible (white-out or severe detachment) |

| | **Structural damage¹** |
|---|---|
| 1 | Retinal detachment with folding |
| 2 | Not visible (white-out or severe detachment) |

| | |
|---|---|
| ¹ Posterior uveitis was ultimately scored without taking into account structural damage as it was not applicable. | |

### RESULTS

### Non-specific clinical observations

From Day -22 until the end of the experiment, animals did not show any non-specific clinical signs.

### Bodyweights

Bodyweights for huMBP and huMBP + IRBP models, expressed as a percentage of the initial (Day -22) bodyweights, were analysed by two-way ANOVA followed by Dunnett's *post*-test for multiple comparisons between experimental groups and vehicle group. Two-way ANOVA revealed a significant effect of time and treatment in both huMBP and huMBP + IRBP models (all p < 0.0001). In the huMBP model, Dunnett's *post*-test revealed a significant increase in bodyweight on Days 22, 24 and 28 in the Cyclosporin A group (all p < 0.05) and in the ATX peptides group (p < 0.05, p < 0.05 and p < 0.01, respectively) *vs*. vehicle group. In the huMBP + IRBP model, Dunnett's post-test revealed a significant increase in bodyweight on Days 13, 15, 22, 24 and 28 in the Cyclosporin A group (p < 0.05, p < 0.05, p < 0.05, p < 0.01 and p < 0.01, respectively) vs. vehicle group.

### Anterior uveitis clinical scores

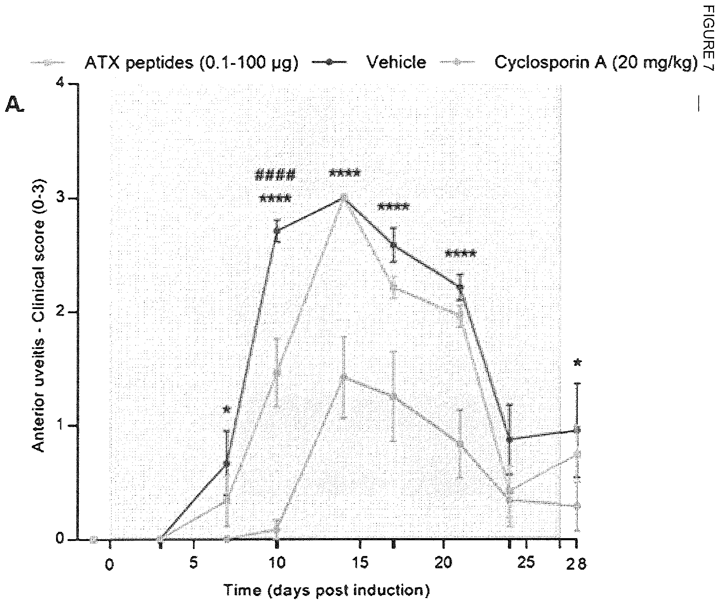

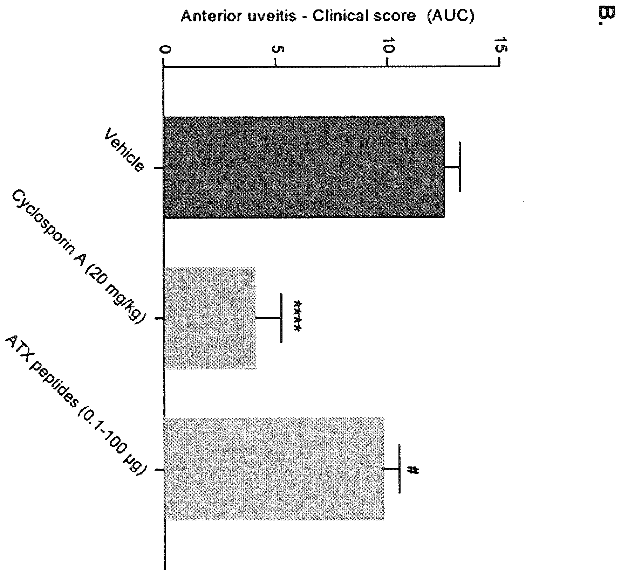

Animals with an average total score (average of left eye plus right eye) of 1 on two consecutive occasions or >1 after any TEFI session were considered to have developed clinical signs of anterior uveitis. Anterior uveitis clinical scores were analysed by two-way ANOVA followed by Dunnett's *post*-test for multiple comparisons between experimental groups and vehicle group. In the huMBP model, all mice displayed no clinical signs of anterior uveitis throughout the course of the study. In the huMBP + IRBP model, a two-way ANOVA revealed a significant effect of time and treatment (both p < 0.0001). A Dunnett's *post*-test revealed a significant improvement in clinical scores in the Cyclosporin A group on Days 7, 10, 14, 17, 21 and 28 (p < 0.05, p < 0.0001, p < 0.0001, p < 0.0001, p < 0.0001 and p < 0.05, respectively) and in the ATX peptides group on Day 10 (p < 0.0001) *vs.* vehicle group (Fig. 7A). An unpaired two-tailed two-sample Student's t-test of area under the curve (AUC) analysis of anterior uveitis clinical score data revealed a significant effect of Cyclosporin A (p < 0.0001) and ATX peptides vs. vehicle (p < 0.05) (Fig. 7B).

### Posterior/intermediate uveitis clinical scores

Animals with an average total score (average of left eye plus right eye) of 1 on two consecutive occasions or >1 after any TEFI session were considered to have developed clinical signs of posterior uveitis. Posterior uveitis clinical scores were analysed by two-way ANOVA followed by Dunnett's *post*-test for multiple comparisons between experimental groups and vehicle group. In the huMBP model, a two-way ANOVA revealed a significant effect of time and treatment (both p < 0.0001). A Dunnett's *post*-test revealed a significant improvement in clinical scores in the Cyclosporin A group on Days 7, 10, 14, 17, 21, 24 and 28 (p < 0.05, p < 0.0001, p < 0.0001, p < 0.0001, p < 0.0001, p < 0.01 and p < 0.05, respectively) and in the ATX peptides group on Days 7, 10, 14, 17, 21, 24 and 28 (p < 0.05, p < 0.0001, p < 0.0001, p < 0.0001, p < 0.0001, p < 0.01 and p < 0.05, respectively) *vs.* vehicle group (Fig. 8A). In the huMBP model, an unpaired two-tailed two-sample Student's t-test of area under the curve (AUC) analysis of posterior uveitis clinical score data revealed a significant effect of Cyclosporin A (p < 0.0001) and ATX peptides (p < 0.0001) vs. vehicle (Fig. 8B).

In the huMBP + IRBP model, a two-way ANOVA revealed a significant effect of time and treatment (both p < 0.0001). A Dunnett's post-test revealed a significant improvement in clinical scores in the Cyclosporin A group on Days 7, 10, 14, 17 and 21 (p < 0.05, p < 0.0001, p < 0.0001, p < 0.0001 and p < 0.05, respectively) and in the ATX peptides group on Day 10 (p < 0.01) vs. vehicle group (Fig. 8C). An unpaired two-tailed two-sample Student's *t*-test of area under the curve (AUC) analysis of posterior uveitis clinical score data revealed a significant effect of Cyclosporin A vs. vehicle (p < 0.0001) (Fig. 8D). An unpaired one-tailed two-sample Student's t-test of area under the curve (AUC) analysis of posterior uveitis clinical score data revealed a significant effect of ATX peptides vs. vehicle (p < 0.05) (Fig. 8D).

### Incidence of posterior/intermediate uveitis

Animals with an average total score (left eye plus right eye) of one on two consecutive occasions or >1 after any TEFI session were considered to have developed clinical signs of posterior uveitis.

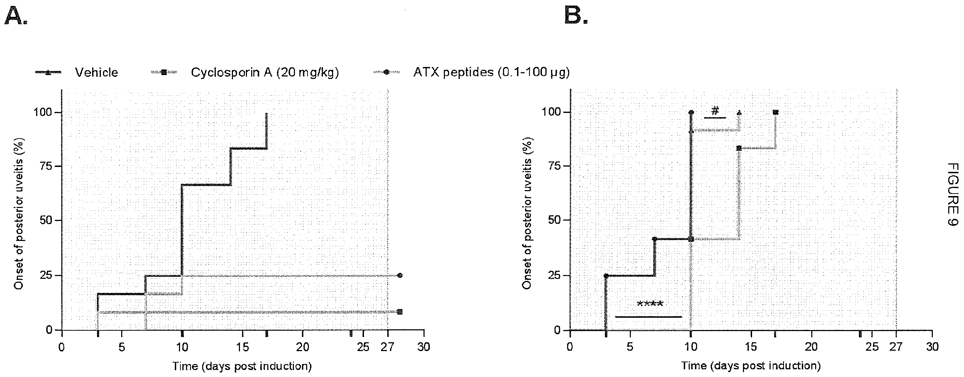

In the huMBP model, animals treated with Cyclosporin A and ATX peptides had reduced incidence compared to vehicle-treated animals (Fig. 9A). Only one animal treated with Cyclosporin A and three animals treated with the ATX peptides went on to develop clinical signs of EAU (see Table 8).

**Table 8**

| **Group** | **Incidence of uveitis** | **Onset (Day)** | **Mean Maximum Score** |
|---|---|---|---|
| huMBP, Vehicle | 100% (12/12) | 10.4 ± 1.3 | 1.8 ± 0.07 |
| huMBP, Cyclosporin A | 5% (1/12) | 3 | 1.2 ± 0.7 |
| huMBP, ATX peptide | 25% (3/12) | 8 ± 1 | 1.08 ± 0.2^{$} |
| huMBP/IRBP, Vehicle | 100% (12/12) | 7.7 ± 0.8 | 7.9 ± 0.08 |
| huMBP/IRBP, Cyclosporin A | 100% (12/12) | 12.8 ± 0.7 | 6.1 ± 0.5 |
| huMBP/IRBP, ATX peptide | 100% (12/12) | 10.3 ± 0.3 | 7.9 ± 0.08 |

| | | | |
|---|---|---|---|
| ^{$} *For animal 3.10, score of 4 (average of left and right eye score) on Day 21 was not included into data analysis (outlier identified analysing Day 21 posterior uveitis clinical score data with Grubb's test with alpha = 0.05)* | | | |

In the huMBP + IRBP model, Cyclosporin A significantly delayed the onset of posterior uveitis if compared to the vehicle-treated group (p < 0.0001) (Fig. 9B). ATX peptides significantly delayed onset of posterior/intermediate uveitis compared to vehicle-treated group (p < 0.05) (Fig. 9B).

### CONCLUSIONS

Administration of huMBP alone or combined with IRBP induced clinical signs of posterior uveitis resulting in enlarged blood vessels, retinal inflammation and cellular infiltration, as expected in these models of EAU. Clinical signs of posterior uveitis were less severe in the huMBP model compared to animals receiving huMBP + IRBP.

Clinical signs of anterior uveitis were observed in the huMBP + IRBP model but not in the huMBP model. In the huMBP + IRBP model, clinical signs of anterior uveitis included increased vascularisation of the iris and cellular infiltration of the anterior chamber as well as abnormal pupil contraction.

Overall, in the huMBP + IRBP model, Cyclosporin A and ATX peptides significantly reduced the clinical signs of anterior uveitis.

In the huMBP model, Cyclosporin A and ATX peptides reduced the incidence of posterior uveitis compared to vehicle-treated animals.

Cyclosporin A and ATX peptides significantly delayed the onset of posterior uveitis in the huMBP + IRBP model, when compared to the vehicle-treated group.

Overall, in the huMBP and huMBP + IRBP models, Cyclosporin A and ATX peptides significantly reduced clinical signs of posterior/intermediate uveitis.

Various modifications and variations of the described methods and system of the invention will be apparent to those skilled in the art. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology, cellular immunology or related fields are intended to be within the scope of the following claims.

## Claims

1. A composition which comprises a myelin basic protein peptide selected from the following:
MBP 30-44, consisting of SEQ ID NO:1;
MBP 83-99, consisting of SEQ ID NO:2;
MBP 131-145, consisting of SEQ ID NO:3; and
MBP 140-154, consisting of SEQ ID NO:4
for use in treating or preventing uveitis in a subject.

2. A composition for use according to claim 1 wherein said composition comprises:
MBP 30-44, consisting of SEQ ID NO:1;
MBP 83-99, consisting of SEQ ID NO:2;
MBP 131-145, consisting of SEQ ID NO:3; and
MBP 140-154, consisting of SEQ ID NO:4.

3. A composition for use according to claim 1, wherein the uveitis is posterior uveitis, anterior uveitis or intermediate uveitis.

4. A kit which comprises a myelin basic protein peptide selected from the following:
MBP 30-44, consisting of SEQ ID NO:1;
MBP 83-99, consisting of SEQ ID NO:2;
MBP 131-145, consisting of SEQ ID NO:3; and
MBP 140-154, consisting of SEQ ID NO:4
for use in the treatment or prevention of uveitis in a subject.

5. A kit for use according to claim 4 wherein said kit comprises
MBP 30-44, consisting of SEQ ID NO:1;
MBP 83-99, consisting of SEQ ID NO:2;
MBP 131-145, consisting of SEQ ID NO:3; and
MBP 140-154, consisting of SEQ ID NO:4.

6. A kit for use according to claim 5 wherein said MBP peptides are administered simultaneously, separately or sequentially.

## Patentansprüche

1. Zusammensetzung, die ein aus dem Folgenden ausgewähltes MBP(Myelin Basic Protein)-Peptid umfasst:
MBP 30-44, bestehend aus SEQ ID NO:1;
MBP 83-99, bestehend aus SEQ ID NO:2;
MBP 131-145, bestehend aus SEQ ID NO:3; und
MBP 140-154, bestehend aus SEQ ID NO:4,
zur Verwendung beim Behandeln oder Vorbeugen von Uveitis bei einem Individuum.

2. Zusammensetzung zur Verwendung gemäß Anspruch 1, wobei die Zusammensetzung Folgendes umfasst:
MBP 30-44, bestehend aus SEQ ID NO:1;
MBP 83-99, bestehend aus SEQ ID NO:2;
MBP 131-145, bestehend aus SEQ ID NO:3; und
MBP 140-154, bestehend aus SEQ ID NO:4.

3. Zusammensetzung zur Verwendung gemäß Anspruch 1, wobei es sich bei der Uveitis um Uveitis posterior, Uveitis anterior oder Uveitis intermedia handelt.

4. Kit, das ein aus dem Folgenden ausgewähltes MBP(Myelin Basic Protein)-Peptid umfasst:
MBP 30-44, bestehend aus SEQ ID NO:1;
MBP 83-99, bestehend aus SEQ ID NO:2;
MBP 131-145, bestehend aus SEQ ID NO:3; und
MBP 140-154, bestehend aus SEQ ID NO:4,
zur Verwendung bei der Behandlung oder Vorbeugung von Uveitis bei einem Individuum.

5. Kit zur Verwendung gemäß Anspruch 4, wobei das Kit Folgendes umfasst:
MBP 30-44, bestehend aus SEQ ID NO:1;
MBP 83-99, bestehend aus SEQ ID NO:2;
MBP 131-145, bestehend aus SEQ ID NO:3; und
MBP 140-154, bestehend aus SEQ ID NO:4.

6. Kit zur Verwendung gemäß Anspruch 5, wobei die MBP-Peptide gleichzeitig, getrennt oder hintereinander verabreicht werden.

## Revendications

1. Composition qui comprend un peptide de protéine basique de la myéline choisi parmi les suivants :
MBP 30 à 44, constitué de la SEQ ID NO: 1 ;
MBP 83 à 99, constitué de la SEQ ID NO: 2 ;
MBP 131 à 145, constitué de la SEQ ID NO: 3 ; et
MBP 140 à 154, constitué de la SEQ ID NO: 4
pour une utilisation dans le traitement ou la prévention d'une uvéite chez un sujet.

2. Composition pour une utilisation selon la revendication 1, ladite composition comprenant :
MBP 30 à 44, constitué de la SEQ ID NO: 1 ;
MBP 83 à 99, constitué de la SEQ ID NO: 2 ;
MBP 131 à 145, constitué de la SEQ ID NO: 3 ; et
MBP 140 à 154, constitué de la SEQ ID NO: 4.

3. Composition pour une utilisation selon la revendication 1, l'uvéite étant une uvéite postérieure, une uvéite antérieure ou une uvéite intermédiaire.

4. Kit qui comprend un peptide de protéine basique de la myéline choisi parmi les suivants :
MBP 30 à 44, constitué de la SEQ ID NO: 1 ;
MBP 83 à 99, constitué de la SEQ ID NO: 2 ;
MBP 131 à 145, constitué de la SEQ ID NO: 3 ; et
MBP 140 à 154, constitué de la SEQ ID NO: 4
pour une utilisation dans le traitement ou la prévention d'une uvéite chez un sujet.

5. Kit pour une utilisation selon la revendication 4, ledit kit comprenant
MBP 30 à 44, constitué de la SEQ ID NO: 1 ;
MBP 83 à 99, constitué de la SEQ ID NO: 2 ;
MBP 131 à 145, constitué de la SEQ ID NO: 3 ; et
MBP 140 à 154, constitué de la SEQ ID NO: 4.

6. Kit pour une utilisation selon la revendication 5, lesdits peptides MBP étant administrés simultanément, séparément ou séquentiellement.
